# EUROPEAN PATENT SPECIFICATION

(11) **EP 2 046 498 B1**
(45) Date of publication and mention of the grant of the patent: **16.11.2011**
(21) Application number: 07789927.6
(22) Date of filing: 30.07.2007
(51) Int. Cl.: B01L 3/00

(54) **A MICROFLUIDIC DEVICE FOR CONTROLLED MOVEMENT OF MATERIAL AND A METHOD FOR DELIVERING A MATERIAL FROM A MICROFLUIDIC DEVICE**
MIKROFLUIDVORRICHTUNG ZUR GESTEUERTEN MATERIALBEWEGUNG UND VERFAHREN ZUR ZUFÜHRUNG VON MATERIAL VON EINER MIKROFLUIDVORRICHTUNG
DISPOSITIF MICROFLUIDIQUE POUR UN MOUVEMENT CONTRÔLÉ D'UN MATÉRIAU ET PROCÉDÉ POUR ACHEMINER UN MATÉRIAU DEPUIS UN DISPOSITIF MICROFLUIDIQUE

(30) Priority: 28.07.2006 US 495359
(43) Date of publication of application: 15.04.2009
(73) Proprietor: Janisys Limited, Galway (IE)
(72) Inventor: ZHANG, Sean, Xiao-An, Palo Alto, CA 94304 (US); BECK, Patricia, A., Palo Alto, CA 94304 (US); NICKEL, Janice, H., Palo Alto, CA 94304 (US)
(74) Representative: Gorman, Francis Fergus
(86) International application number: PCT/IE2007/000074
(87) International publication number: WO 2008/012788

(56) References cited:
- WO-A-2005/016558
- US-A1- 2004 248 326
- US-A1- 2005 038 379
- US-A1- 2007 104 023
- HILT ET AL: "Microfabricated drug delivery devices" INTERNATIONAL JOURNAL OF PHARMACEUTICS, AMSTERDAM, NL, vol. 306, no. 1-2, 8 December 2005 (2005-12-08), pages 15-23, XP005173572 ISSN: 0378-5173

## Description

The present invention relates to a microfluidic device for controlled movement of material, and the invention also relates to a method for delivering a material from a microfluidic device.

There has been a growing interest in the use of microfluidic systems in chemical and biological sciences, medical specialities, as well as manufacturing and dispensing operations. This interest has been attributable to the potential increased performance of relatively complicated chemical and biochemical systems which use relatively small volumes of fluids. Microfluidic systems have been employed in these types of applications to introduce the fluids because such systems allow for more easily measured reactions. In addition, minimising sample volumes have resulted in lowered reagent costs, less toxic material-introduction and more easily modelled reactions.

Microfluidic systems for drug delivery have until now used a contacting, but non-penetrating, patch, with or without enhancing agents, to move drugs diffusively through the skin. For those microfluidic systems that use a pump, the actuation unit is typically rather bulky in construction because the actuation units are oftentimes based upon conventional actuating devices, such as piezoelectric and thermoelectric actuators. Many fluids of interest, including drugs, however, are incapable of being delivered in precise extremely low dose amounts through use of these types of actuating devices, necessitating delivery of a larger volume of fluid for reliability and repeatability of the actuation unit. In addition, many drugs may become damaged or otherwise rendered unfit for their intended purposes through use of conventional actuating units.

PCT Application Publication No. WO 2005/016558 of COPPETA et al discloses a method and a device for selective release of a drug from a sealed reservoir. The device of COPPETA comprises a holding cavity for the drug, and a cavity for a thermally expandable activation material which is separated from the holding cavity by an actuation pin. Heating of the thermally expandable activation material urges the actuation pin into the holding cavity to discharge the drug from the holding cavity.

There is therefore a need for a microfluidic device which addresses at least some of these problems.

The present invention is directed towards providing such a microfluidic device, and the invention is also directed towards providing a method for delivering a material from a microfluidic device.

According to the invention there is provided a microfluidic device for controllably moving a material of interest, said microfluidic device comprising a holding cavity for holding the material of interest, an activation material configured to expand, wherein expansion of the activation material decreases the size of the holding cavity to thereby cause the material of interest to be released from the holding cavity, and at least one actuator configured to induce the activation material to expand, wherein the activation material comprises a gas configured to remain in a dissolved state at relatively lower temperatures and to evolve back into a gaseous state at relatively higher temperatures, and the at least one actuator is operable on the activation material at multiple levels between a zero induction level whereby the activation material is maintained in the dissolved state, and a maximum induction level to controllably cause the activation material to evolve from the dissolved state to the gaseous state to decrease the size of the holding cavity to release a specified volume of the material of interest therefrom.

Preferably, the microfluidic device further comprises at least one delivery orifice configured for fluid communication with the holding cavity.

In one embodiment of the invention the at least one delivery orifice comprises a hydrophobic needle.

In another embodiment of the invention the microfluidic device further comprises at least one of a barrier positioned between the at least one delivery orifice and the holding cavity.

In a further embodiment of the invention the material of interest is configured to flow through one or both of the at least one delivery orifice and the barrier when the activation material one of expands and contracts.

In one embodiment of the invention the at least one actuator is configured to increase the temperature of the activation material to evolve the activation material back into the gaseous state and thereby cause the material of interest to be released through the at least one delivery orifice.

In one embodiment of the invention the activation material comprises at least one of water, alcohol and ammonia.

In another embodiment of the invention the microfluidic device further comprises:
a space configured to hold at least one of a solvent and water; and
a membrane separating the holding cavity and the space;
the at least one actuator being configured to induce the activation material to expand, thereby causing the membrane to break and enabling the material of interest and the at least one of the solvent and the water to mix prior to being moved out of the microfluidic device.

In another embodiment of the invention the microfluidic device further comprises an actuation cavity housing the activation material, wherein the actuation cavity is separated from the holding cavity by a flexible membrane.

In an alternative embodiment of the invention the material of interest is interspersed with the activation material. Preferably, expansion of the activation material causes the material of interest and the activation material to be released through the delivery orifice. Advantageously, the material of interest is substantially coated with a substance configured to substantially separate the material of interest from the activation material. Ideally, the substance substantially coating the material of interest is water insoluble and removable by an enzyme.

In one embodiment of the invention the activation material comprises at least one of di-methyl ether and ethyl methyl ether.

Preferably, the microfluidic device further comprises:
a power source for powering the at least one actuator; and
a controller for controlling delivery of power to the at least one actuator.

Advantageously, the power source and the controller are integrally formed with the microfluidic device.

In one embodiment of the invention the at least one actuator comprises a resistive element configured to become heated through application of a potential difference, allowed by the controller.

In another embodiment of the invention the material of interest is provided in a form which is soluble in a suitable solvent.

In a further embodiment of the invention the material of interest is in the form of a reactant. Preferably, the material of interest is in a pure form.

Preferably, the material of interest is reconstituted in the holding cavity. Advantageously, the material of interest is reconstituted by a solvent. Preferably, the material of interest is reconstituted by contact with the solvent. Ideally, the material of interest is reconstituted by mixing with the solvent.

In another embodiment of the invention the at least one actuator is operable to intersperse the solvent with the material of interest for reconstitution thereof. Preferably, the at least one actuator is operable to intersperse the solvent with the material of interest for reconstitution thereof and to subsequently act on the activation material for releasing the reconstituted material of interest from the holding cavity. Advantageously, the at least one actuator is operable to provide a time delay between the time the solvent is interspersed with the material of interest and the activation material is subsequently acted on for releasing the reconstituted material of interest from the holding cavity. Ideally, the time delay is of sufficient duration for facilitating reconstitution of the material of interest.

In one embodiment of the invention the solvent is held in a holding cavity separate from the holding cavity for holding the material of interest.

Preferably, the solvent comprises a reconstituting amount of the activation material.

In another embodiment of the invention the solvent is provided by the activation material.

In one embodiment of the invention the material of interest is freeze dried.

In another embodiment of the invention the activation material comprises carbon dioxide.

In a further embodiment of the invention the holding cavity comprises dimensions ranging between micron to millimetre scales.

The invention also provides a method for delivering a material of interest from a microfluidic device having at least one cavity and a delivery orifice, said method comprising:
dissolving a gaseous activation material at a relatively low temperature;
inserting the dissolved gaseous activation material into the at least one cavity of the microfluidic device;
maintaining the dissolved gaseous activation material at a relatively low temperature; and
heating the dissolved gaseous activation material to cause the dissolved gaseous activation material to evolve into a gaseous state and expand, expansion of the gaseous activation material causing the material of interest to be delivered out of the delivery orifice.

In another embodiment of the invention the microfluidic device includes a holding cavity and an actuation cavity separated by a flexible membrane, and inserting the dissolved gaseous activation material comprises inserting the dissolved gaseous activation material into the actuation cavity.

In an alternative embodiment of the invention the method further comprises:
combining the material of interest and the dissolved gaseous activation material into a mixture; and
inserting the mixture into the at least one cavity.

Preferably, heating the dissolved gaseous activation material further comprises heating the mixture of the material of interest and the dissolved gaseous activation material.

The Invention will be more dearly understood from the following description of some preferred embodiments thereof, which are given by way of example only, with reference to the accompanying drawings, in which:
Fig. 1A shows a block diagram of a microfluidic device for controlled release of material, according to an embodiment of the invention,
Fig. 1B shows a schematic diagram, partially in cross-section, of a part of a microfluidic device, according to an embodiment of the invention,
Fig. 1C shows a schematic diagram similar to Fig. 1B, where an activation system in the microfluidic device has expanded,
Fig. 1D shows a schematic diagram, partially in cross-section, of a part of a microfluidic device, according to another embodiment of the invention,
Fig. 1E shows a schematic diagram, partially in cross-section, of a part of a microfluidic device, according to a further embodiment of the invention,
Fig. 2 illustrates a graph depicting the amount of carbon dioxide gas evolved as a function of temperature,
Figs. 3 and 4 depict flow diagrams of respective methods for delivering a material from a microfluidic device, according to two embodiments of the invention, and
Fig. 5 is a block diagram illustrating a computer system or other smart device operable to perform one or more functions on a microfluidic device, according to an embodiment of the invention.

For simplicity and illustrative purposes, the principles of the embodiments of the invention are described by referring mainly to examples thereof. In the following description, numerous specific details are set forth in order to provide a thorough understanding of the embodiments. It will be apparent, however, to one of ordinary skill in the art that the embodiments may be practised without limitation to these specific details. In other instances, well known methods and structures have not been described in detail so as not to unnecessarily obscure the embodiments.

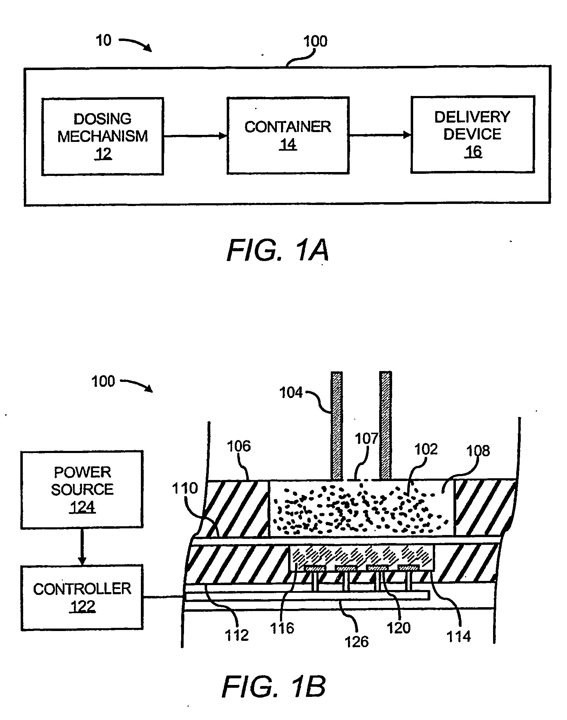

With reference first to Fig. 1A, a block diagram 10 of a microfluidic device 100 for controlled release of material is depicted, according to an example. It should be understood that the following description of the block diagram is but one manner of a variety of different manners in which such a microfluidic device 100 may be configured. In addition, it should be understood that the microfluidic device 100 may include additional components and that some of the components described herein may be removed and/or modified without departing from a scope of the microfluidic device 100. For instance, the microfluidic device 100 may include any number of sensors, memories, processors, air moving devices, vent tiles, etc., as well as other components, which may be implemented in the operations of the microfluidic device 100.

As shown in the block diagram 10 of Fig. 1A, the microfluidic device 100 includes a dosing mechanism 12, a container 14, and a delivery device 16. The dosing mechanism 12 may include a plunger or other actuation device capable of applying sufficient force on a material of interest contained in the container 14, such that the material of interest is released or received through the delivery device 16. In addition, the dosing mechanism 12 may operate to controllably release the material of interest from or receive the material of interest into the container 14, through the delivery device 16. It should further be noted that the dosing mechanism 12 may, in some instances, be a sampling mechanism configured to apply sufficient negative force to collect a material of interest through the delivery device 16.

The container 14 may include any reasonably suitable container for containing the material of interest and the delivery device 16 may include, for instance, a needle, an orifice, a tube and the like.

A more detailed description of the elements forming the microfluidic device 100 is provided herein below with respect to Figs. 1B to 1E.

With reference first to Fig. 1B, there is shown a schematic diagram, partially in cross-section, of a part of the microfluidic device 100, according to an example. It should be readily apparent that the microfluidic device 100 depicted in Fig. 1B represents a generalised illustration and that other features may be added or existing features may be removed or modified without departing from a scope of the microfluidic device 100. For example, the microfluidic device 100 may include additional features as discussed herein below.

Generally speaking, the microfluidic device 100 may be employed to controllably deliver a material of interest 102, such as a drug, reagent or other type of material. More particularly, for instance, the microfluidic device 100 may be employed to inject or otherwise supply the material of interest 102 onto or through a surface, such as vial membrane or skin. In addition; or alternatively, the microfluidic device 100 may be employed to collect a sample of a specific volume of the material of interest 102. In any regard, the microfluidic device 100 may include a plurality of delivery orifices 104, which may include, for instance, microneedles, tubes, etc. In Fig. 1B. a single delivery orifice 104 is depicted for purposes of simplicity. It should be understood, however, that the microfluidic device 100 may include any reasonably suitable number of delivery orifice 104.

As shown in Fig. 1B, the delivery orifice 104 is attached to a first spacer layer 106. The first spacer layer 106 may include any reasonably suitable material, such as silicon, glass, polymers, ceramics, etc. The first spacer layer 106 may be formed with a plurality of holding cavities 108 configured to house some or all of the material of interest 102. In addition, the holding cavities 108 may be associated with respective delivery orifices 104, such that the material of interest 102 contained in one of the holding cavities 108 may be delivered through one or more delivery orifices 104. It should be readily understood that a single holding cavity 108 is depicted in Fig. 1B for purposes of simplicity and not of limitation.

The microfluidic device 100 is also depicted as including an optional barrier 107 between the holding cavity 108 and the delivery orifice 104. The optional barrier 107 may generally operate to substantially prevent the material of interest 102 from being prematurely released through the delivery orifice. In addition, the optional barrier 107 may enable the material of interest 102 to flow into the delivery orifice 104 when sufficient pressure is applied on the material of interest 102 contained in the holding cavity 108. As such, the optional barrier 107 may include one or more openings configured to open or otherwise accommodate fluid movement when a sufficient amount of pressure is applied on the material of interest 102. In addition, or alternatively, the optional barrier 107 may be configured to rupture or otherwise create an opening when a sufficient amount of pressure is applied on the material of interest 102.

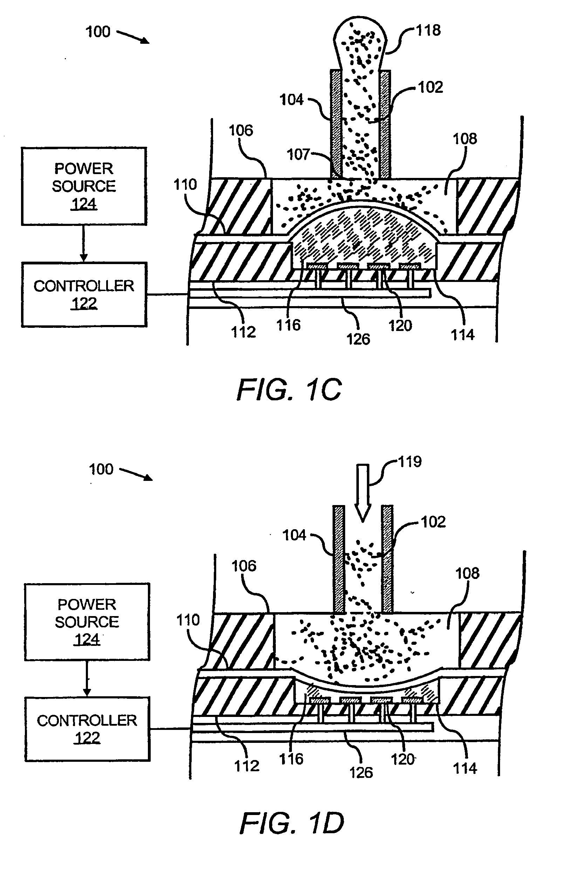

The barrier 107 is considered optional because the microfluidic device 100 may operate properly in certain instances without the use of the barrier 107. For instance, the delivery orifice 104 may include a hydrophobic needle capable of preventing delivery of the material of interest 102 until it is desired to do so, which therefore removes the barrier 107 requirement. It should, however, be understood that the barrier 107 may be used in conjunction with the hydrophobic needle without departing from a scope of the microfluidic device 100. In addition, the barrier 107 may be omitted, for instance, when the delivery orifice 104 includes a hydrophobic needle or when the material of interest 102 may otherwise remain within the holding cavity 108 without the use of the barrier 107, when the barrier 107 is not required to shield the material of interest 102, when the microfluidic device 100 is employed to collect samples of the material of interest 102, as shown in Fig. 1D, etc.

A bottom section of the holding cavity 108 is depicted as being formed by a membrane 110. As discussed in greater detail herein below, the membrane 110 includes a flexible membrane configured to change the size of the holding cavity 108. First, however, a discussion of a second spacer layer 112 is provided. The second spacer layer 112 may include the same material as the first spacer layer 106. Alternatively, however, the second spacer layer 112 may include a different material from the first spacer layer 106, and may include any of the materials listed above with respect to the first spacer layer 106.

According to an example, the first spacer layer 106 and the second spacer layer 112 may include a single component. In this example, the membrane 110 may be formed as part of the first spacer layer 106 and the second spacer layer 112, through, for instance, an etching process. In another example, the first spacer layer 106, the second spacer layer 112 and the membrane 110 may include separate elements that are bonded together.

In any regard, the second spacer layer 112 may include one or more actuation cavities 114 configured to house an activation material 116. The activation material 116 is operable to expand and cause the membrane 110 to deflect, thereby causing the material of interest 102 to be released through the delivery orifice 104. Various examples of suitable activation materials 116 are described herein below. In addition, although a single actuation cavity 114 is depicted in Fig. 1B, it should be understood that the second spacer layer 112 may include any reasonably suitable number of actuation cavities 114 without departing from a scope of the microfluidic device 100 disclosed herein. The actuation cavity 114 may moreover include multiple compartments.

As shown in Fig. 1B, a top section of the actuation cavity 114 may be formed by the membrane 110. The membrane 110 generally includes a flexible membrane configured to separate the activation material 116 from the material of interest 102 and to increase the size of the actuation cavity 114 as the activation material 116 expands, as shown in greater detail in Fig. 1C. More particularly, and as shown in Fig. 1C, expansion of the activation material 116 causes the membrane 110 above the actuation cavity 114 to deflect in a direction toward the delivery orifice 104. If the deflection of the membrane 110 also reduces the size of the holding cavity 108 for the material of interest 102, the material of interest 102 is then released from the delivery orifice 104, as shown as a released portion 118 of the material of interest 102.

According to another example, and as shown in Fig. 1D, the microfluidic device 100 may operate to draw a material of interest 102 into the holding cavity 108. In Fig. 1D. the activation material 116 is configured to decrease in size, thereby causing the membrane 110 to be deflected away from the delivery orifice 104 and the holding cavity 108 to increase in size. The increase in size of the holding cavity 108 generally causes a negative pressure to be created in the holding cavity 108, which causes the material of interest 102 to be drawn in through the delivery orifice 104, as indicated by the arrow 119.

The activation material 116 may, for instance, include EXPANCEL microspheres available from Expancel, Inc., having an office located in Duluth, Georgia, USA. In this example, the activation material 116 may be in its initial state and may expand when subjected to the appropriate amount of heat, thereby releasing the material of interest 102 through the delivery orifice 104; alternatively, the activation material may be in a fully expanded state and may shrink when subjected to slightly higher heat, thereby drawing the material of interest 102 through the delivery orifice 104. The activation material 116 may also include hydrogels, which may be engineered to either expand or shrink in volume when heated above a threshold temperature or when subjected to a threshold pH level. Further examples of suitable activation materials 116 may include NH₃-water, CO₂-water, etc. The pH levels of these activation materials 116 may change through application of heat. For instance, application of heat on the activation material 116 including the CO₂-water will increase the pH of its solution as more and more CO₂ escapes from the solution. In addition, application of heat on the activation material 116 including the NH₃-water will decrease the pH of its solution as more and more NH₃ escapes out of the solution. CO₂ and NH₃ are two examples of materials that can change pH with application of temperature. It should be understood, however, that other materials having this property, which may be known to those skilled in the art, may also be employed. Additional examples of suitable activation materials 116 include polyvinylchloride with one or more polyesters configured to shrink with applied heat, such as shrink-wrap, shrink-tubing, etc.

In another example, an external vacuum system may be employed in addition to the activation material 116 to create the negative pressure in the holding cavity 108.

The microfluidic device 100 may be fabricated through any suitable fabrication process. For instance, the microfluidic device 100 may include a silicon or glass substrate and photolithography may be implemented to define the holding and actuation cavities 108 and 114. As another example, the microfluidic device 100 may include silicon, plastic or other polymeric material, and moulding steps may be implemented to fabricate the microfluidic device 100. In addition, various combinations of etching, deposition, lithographic formation, moulding, stamping, imprinting, etc. Processes may be employed to fabricate the microfluidic device 100.

With reference back to Fig. 1B, also shown within the actuation cavity 114 are a plurality of actuators 120. As described in greater detail herein below, the actuators 120 may include various types of actuators and may be operated to controllably expand or contract the activation material 116. Although a plurality of actuators 120 have been illustrated in Fig. 1B, it should be understood that a single actuator 120 may be provided in the actuation cavity 114 without departing from a scope of the microfluidic device 100. In addition, the actuation cavity 114 may contain liquids, gels, solids, vapours, or a combination thereof, which may assist in the expansion or contraction of the activation material 116.

In any regard, the actuators 120 may be controlled on-board, for instance, through a conductive line 126, or remotely by a controller 122, which may include a microprocessor, a microcontroller, an application specific integrated circuit (ASIC), sensors, feedback devices and the like, configured to perform various processing functions. In addition, or alternatively, the controller 122 may include software operating in one or more computing devices. Furthermore, either or both of the controller 122 and a power source 124 may be integrally formed with the microfluidic device 100 or they may include devices that are separate from the microfluidic device 100.

In conjunction with the power source 124, the controller 122 may be configured to activate the actuators 120 according to one or more control schemes. For instance, the controller 122 may be configured to allow actuators 120 to be addressed in response to a local or remote command (for instance, through the push of an activation button or through receipt of a wireless signal). As another example, the controller 122 may be programmed to activate the actuators 120 at a predetermined time, or at predetermined intervals of time, etc. In addition, or alternatively, the controller 122 may be programmed to activate a single actuator, or one or more sets of actuators 120 to deliver the material of interest 102 from a first set of holding cavities 108 at a first time and to activate another set of actuators 120 to deliver the material of interest 102 from a second set of holding cavities 108 at a second time.

In addition, or alternatively, the controller 122 may be programmed to vary operations of the actuators 120 such that the actuators 120 may be operated at multiple levels between a zero induction level and a maximum induction level on the activation material 116 to thereby controllably expand or contract the holding cavity 108. As such, the controller 122 may control the amounts of the material of interest 102 that are released from or received into the holding cavity 108 at various amounts between a zero amount and a maximum amount.

In one example, the controller 122 may vary the number of actuators 120 that are activated to thereby vary the induction levels applied on the activation material 116. In this example, the controller 122 may be programmed with data indicating the number of actuators 120 required to be activated in order to release a predetermined amount of the material of interest 102. For instance, the controller 122 may be programmed to activate two of the actuators 120 to release 30% of the material of interest 102, to activate three of the actuators 120 to release 50% of the material of interest 102, etc. Likewise, the controller 122 may be programmed to activate two of the actuators 120 to receive an amount of the material of interest 102 that fills 30% of the holding cavity 108, to activate three of the actuators 120 to receive an amount of the material of interest 102 that fills 60% of the holding cavity 108, etc.

In another example, the controller 122 may control the power supplied to the actuators 120. More particularly, for instance, the controller 122 may control the temperatures of the actuators 120 at multiple levels between a zero temperature change to a maximum temperature change, where a maximum temperature change is configured to expand or contract substantially all of the activation material 116. Additionally, the controller 122 may control the temperature changes of the actuators 120 for desired periods of time. For instance, the controller 122 may achieve a first temperature for a first period of time, a second temperature for a second period of time, a third temperature for a third period of time, etc. In this regard, the controller 122 may control the actuators 120 to deliver or receive the material of interest 102 in a multiple dose manner. In this example, the controller 122 may be programmed with data that indicates the amount of time the actuators 120 are required to receive energy to achieve the desired amount of movement of the material of interest 102, the amount of power the actuators 120 are required to receive to achieve the desired amount of movement, etc.

In any of the above examples, the microfluidic device 100 may include sufficiently small dimensions such that the microfluidic device 100 is capable of substantially accurately releasing or receiving pL, nL and µl volumes. By way of example, the holding cavity 108 may include dimensions ranging between millimetre to micron scales, for instance, 1mm x 1mm x 1mm, 500µm x 500µm x 500µm, etc. In addition, the microfluidic device 100 may be manufactured through, for instance, imprinting, stamping, roll-to-roll processes as well as on discrete substrates and/or with more conventional lithographic processes, etc.

The controller 122 may be configured to instruct specific reservoirs to deliver one or more different types of materials at one or more different times. In one example, the controller 122 may be configured to control the delivery of a plurality of drugs based upon different delivery schedules and doses. In this example, the amount of material of interest 102 delivered may be controlled through control of the individual or sets of actuators 120 positioned in the various reservoirs as described above.

According to another example, the actuators 120 may be controllably activated to thereby create a desired level of expansion or contraction of the activation material 116 in a specific reservoir. In this example, the controller 122 may substantially accurately regulate the amount of the material of interest 102 released from or received by the specific reservoir by controlling the expansion or contraction of the activation material 116 in the specific reservoirs.

In one example, the actuators 120 may include resistive elements which experience a temperature rise in response to applied voltage. The resistive elements comprising the actuators 120 may differ from resistive elements employed in conventional thermal inkjet systems. For instance, in conventional thermal inkjet systems, the resistive elements are configured to heat up very rapidly and thereby cause a substance, such as ink, to vaporise and form a bubble. As the bubble expands, some of the substance/ink is expelled from a holding chamber. Once the bubble collapses, a vacuum is created which draws more of the substance/ink into the holding chamber from a reservoir. The holding chamber is refilled with the substance and this process is repeated, but it is repeated under a single set of parameters. Any total fluid expelled is a quantised multiple of the number of times the system is fired. As such, simply holding the system at temperature longer does not expel more fluid.

The operating conditions useful for thermal inkjet typically induce damaging cavitation, creating spots of very high temperature and shock. In contrast, the resistive elements comprising the actuators 120 are configured to heat up relatively slower or in multiple stages. It should be noted that boiling is not the same as cavitation. The microfluidic device 100 disclosed herein is designed to operate without cavitational effects and to operate in an analogue fashion (different predetermined amount of the material of interest 102 may be expelled from or received through the delivery orifice 104 at one initiation). In this regard, the amounts of the material of interest 102 that are released or received may be controlled with relatively greater degrees of accuracy and control as compared with the use of thermal inkjet systems.

In this example, the activation material 116 may include a material configured to expand or contract when heated. For instance, the activation material 116 may include a liquid having a sufficiently low boiling point temperature such that the activation material 116 is vaporised through application of heat from the actuators 120. In addition, or alternatively, the activation material 116 may include a solid or a gel configured to expand through application of heat, such as the EXPANCEL microspheres discussed above. The activation material 116 may also include hydrogels engineered to expand or contract after reaching a threshold temperature or when subjected to a threshold pH level.

According to an example, the material of interest 102 contained in the holding cavity 108 may be processed to be highly water soluble to enable two-stage delivery of the material of interest 102. For instance, a reactant material may be kept in a separate holding cavity and maintained in solid form and just prior to release, or as part of the release, the reactant material may be mixed with a solvent/water to liquefy it or to place it in solution. By way of example, the reactant material may include a freeze-dried material in an extremely pure form, which may be made into a water-free powder that instantaneously goes into solution when brought in contact with the solvent/water. In this example, the freeze-dried material and the solvent/water may be housed in holding cavities 108 that are separated by a membrane configured to break when the activation material 116 is activated.

As another example, the freeze-dried material may be adhered or otherwise contained in the delivery orifice 104, such that the freeze-dried material may be mixed with the solvent/water as the solvent/water is expelled from the holding cavity 108.

The actuators 120 may be operable to initially intersperse the solvent with the reactant material for reconstitution thereof, and then subsequently act on the activation material to expel the reconstituted material from the holding cavity 108. Further, the actuators 120 may be operated with a time delay so that the actuators 120 are initially operated to intersperse the solvent with the reactant material, and after a time period sufficient to allow reconstitution of the reactant material, the actuators 120 are operated to act on the activation material to expel the reconstituted material from the holding cavity 108.

The reactant material, the solvent and the activation material may be held in separate holding cavities. Alternatively, the activation material may be provided in the form of the solvent, and in which case, the reactant material and the activation material would be held in separate holding cavities. Where the activation material is provided in the form of the solvent, the actuators 120 would be initially operated to intersperse some of the activation material with the reactant material to produce the reconstituted material, and the amount of the activation material interspersed with the reactant material would be a reconstituting amount of the activation material. In these cases, where the reactant material and the solvent and/or the activation material are held in separate holding cavities, the holding cavities would be selectively communicable, and in general, would be communicable in response to operation of the actuators 120. Depending on the type of actuators 120, only some of the actuators 120 may be initially operable to intersperse the solvent or the activation material with the reactant material, as the case may be, and then others of the actuators 120 would be operable to act on the activation material to expel the reconstituted material from the holding cavity 108.

The activation material 116 may additional, or alternatively, include a chemical configured to expand by evolving into a gas through receipt of a current. In this example, the actuators 120 may include devices configured to apply a current through the activation material 116 to thereby cause dissociation of the activation material 116 and expansion of the actuation cavity 114. By way of example, the activation material 116 may include a material configured to expand when heated. Examples of suitable activation materials 116 include ethyl alcohol, isopropyl alcohol, etc. In addition, the activation material 116 may include carbon dioxide in water or ammonia in water.

In another example, the microfluidic device 100 may be configured to deliver a mixture 130 of the activation material 116 and the material of interest 102. In this example, expansion of the activation material 116 may cause both the material of interest 102 and the activation material 116 to be released from the microfluidic device 100. In one regard, therefore, the activation material 116 may include a relatively inert material that does not substantially affect the material of interest 102 nor the target into which the mixture 130 is delivered.

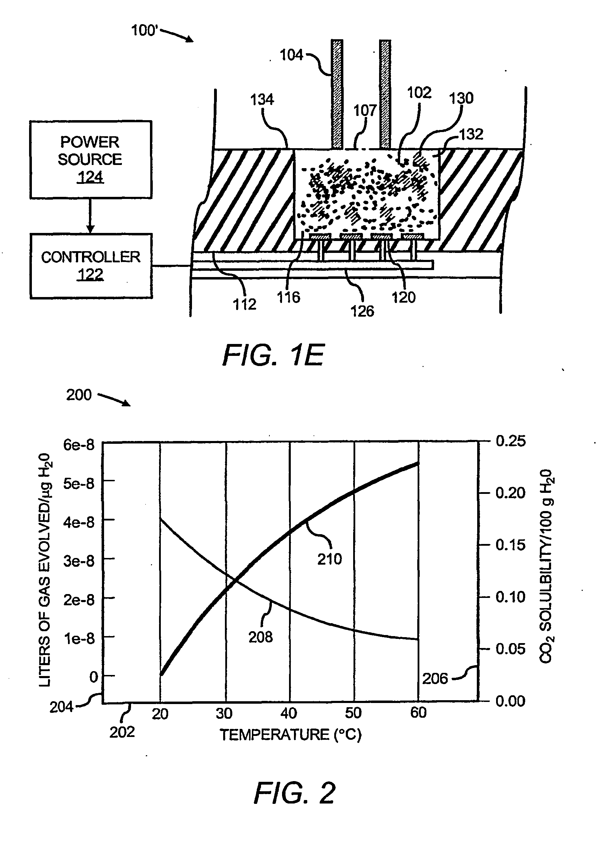

An example of a microfluidic device 100' configured to deliver the mixture 130 of the activation material 116 and the material of interest 102 is shown in Fig. 1E. Many of the elements depicted in Fig. 1E have the same reference numerals as those depicted in Figs. 1B and 1C. It should be understood that those elements that those elements that share the same reference numerals are the same in all of the figures and thus a detailed discussion of those elements is omitted with respect to Fig. 1E. Instead, those elements in Fig. 1E that differ from the elements shown in Figs. 1B and 1C are described with respect to Fig. 1E.

As shown, the microfluidic device 100' includes a single chamber 132 that houses the mixture 130. The single chamber 132 may be formed in a layer 134 of silicon, glass, plastic, polymeric material, etc. In addition, as the activation material 116 expands, the mixture 130 is forced out of the delivery orifice 104. In one regard, therefore, the mixture 130 may contain a sufficient amount of activation material 116 to generally cause a sufficient amount of pressure inside the chamber 132 to cause a desired amount of the material of interest 102 to be released through the delivery orifice 104.

In one example, the activation material 116 may include a dissolved gas configured to remain in the dissolved state at a relatively lower temperature and is configured to return to the gaseous state around a relatively higher temperature. An example of a suitable activation material 116 includes carbon dioxide. More particularly, for instance, carbon dioxide may be dissolved in water within a pH range of around 4 to 9 and at a relatively lower temperature, and the dissolved carbon dioxide may be distributed with a material of interest 102 to form the mixture 130. The gas capture is illustrated in the following examples:

CO₂ + H₂O => H₂CO₃

NH₃ + H₂O => NH₄ ⁺OH

Moreover, the mixture 130 may be expanded through application of heat through the actuators 120 as described above. A graphical representation of how carbon dioxide may be employed as the activation material 116 is depicted in Fig. 2. More particularly, depicted in Fig. 2 is a graph 200 illustrating the amount of carbon dioxide gas evolved as a function of temperature, assuming a water volume of 1pl. Horizontal axis 202 of the graph 200 shows the temperature in degrees Celsius. Vertical axis 204 shows litres of gas evolved per µg of water (H₂O). Vertical axis 206 shows the solubility of carbon dioxide (CO₂) per 100g of water (H₂O). In addition, the thinner line 208 indicates the grams of carbon dioxide dissolved per 100 grams of water at various temperatures and the thicker line 210 indicates the litres of gas evolved over a temperature of 20°C.

As shown in Fig. 2, a relatively large amount of gas may be evolved from a relatively small amount of dissolved carbon dioxide. In this regard, carbon dioxide may be suitable for use as the activation material 116 in the microfluidic device 100'. In addition, the dissolved carbon dioxide may be employed as the activation material 116 in the microfluidic device 100 depicted in Figs. 1B and 1C.

According to another example, the material of interest 102 may be combined in the activation material 116 and may be coated with a material (not shown) configured to protect the material of interest 102 from the activation material 116. In this example, the coating material may include a water insoluble, but enzyme removable material, such as polypeptides, gelatine, starch, etc. Following injection/insertion of the material of interest 102, the coating may be stripped away by a reagent, bodily fluids, etc., which would make the material of interest 102 available for use in the system into which it was introduced.

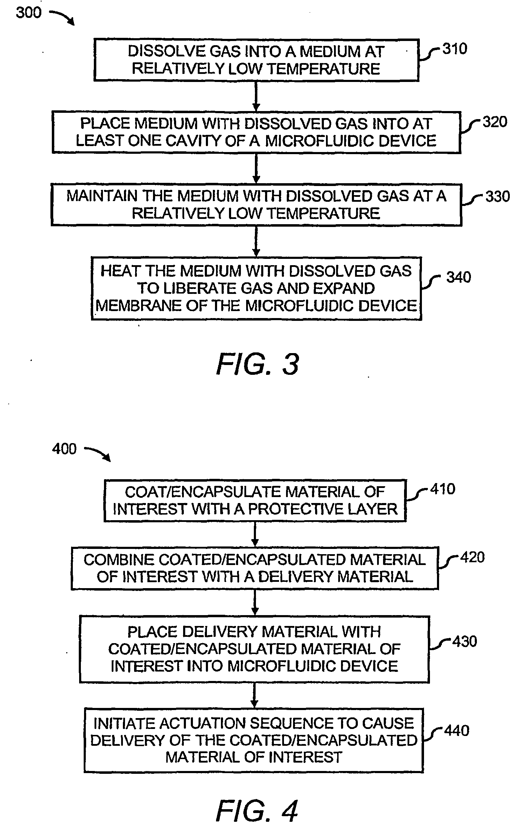

Figs. 3 and 4 show flow diagrams of respective methods 300 and 400 for delivering a material of interest from a microfluidic device, according to two examples. It is to be understood that the following description of the methods 300 and 400 are but two manners of a variety of different manners in which examples of the invention may be practised. It should also be apparent to those of ordinary skill in the art that the methods 300 and 400 represent generalised illustrations and that other steps may be added or existing steps may be removed, modified or rearranged without departing from the scopes of the methods 300 and 400.

The descriptions of the methods 300 and 400 are made with reference to the microfluidic devices 100, 100' illustrated in Figs. 1A to 1E, and thus makes reference to the elements cited therein. It should, however, be understood that the methods 300 and 400 are not limited to the elements set forth in the microfluidic devices 100, 100'. Instead, it should be understood that the methods 300 and 400 may be practised by a microfluidic device having a different configuration than that set forth in the microfluidic devices 100, 100' depicted in figs. 1A to 1E.

With particular reference first to Fig. 3, a gaseous activation material 116 is dissolved at a relatively low temperature at step 310. The gaseous activation material 116 may include carbon dioxide, ammonia, di-methyl ether, methyl ether or any water soluble or partial soluble gaseous chemicals, etc. For instance, carbon dioxide may be dissolved at a temperature according to the graph 200 depicted in Fig. 2.

At step 320, the dissolved gaseous activation material 116 may be inserted into at least one of the holding and actuation cavities 108, 114 of the microfluidic device 100, 100'. In a first example, the dissolved gaseous activation material 116 may be inserted into the actuation cavity 114 as illustrated in Fig. 1B to thereby maintain a separation between the dissolved gaseous activation material 116 and the holding cavity 108. In a second example, the dissolved gaseous activation material 116 may be combined with the material of interest 102 to form a mixture 130 and the mixture 130 may be housed in single chamber 132 of the microfluidic device 100' depicted in Fig. 1E.

In either example, the dissolved gaseous activation material 116 housed in the microfluidic device 100, 100' may be maintained at a relatively low temperature as indicated at step 330. Again, the relatively low temperature may be selected according to the correlations depicted in the graph 200, or any other temperature below the threshold of harm to the material of interest 102 or the maximum temperature of any part or subject in the delivery path.

In addition, at step 340, the dissolved gaseous activation material 116 may be heated to thereby evolve the gaseous activation material 116 back into a gaseous state and expand, where expansion of the gaseous activation material 116 causes the material of interest 102 to be released from the microfluidic device 100, 100'. With reference now to Fig. 4, the material of interest 102 is coated with a protective layer comprising a substance that is water insoluble and removable by an enzyme at step 410. in addition, the coated material 102 may be immersed into an activation material 116 to form a mixture 130 at step 420. At step 430, the mixture 130 may be placed into a cavity 132 of the microfluidic device 100', as shown in Fig. 1E.

At step 440, an actuation sequence may be initiated to cause delivery of the coated material of interest 102. In the actuation sequence, an actuator 120 may be activated to cause the activation material 116 to expand, where expansion of the activation material 116 forces the activation material 116 and the coated material of interest 102 to be delivered from the microfluidic device 100'.

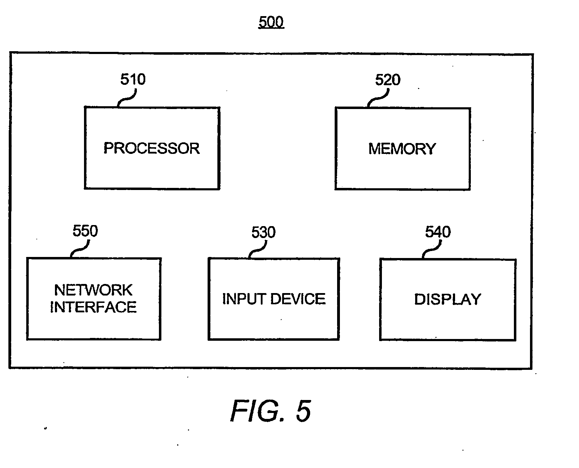

Referring to Fig. 5, a schematic diagram of a computer system 500 is shown in accordance with an embodiment. The computer system or other smart device 500 shown may be used as the controller 122 in the microfluidic devices 100, 100' shown in Fig. 1A to 1E. In one regard, computer system or other smart device 500 may be configured to receive various inputs and to control operations of the actuators 120 to thereby control delivery of the material 102 from the microfluidic devices 100, 100'. By way of example, system 500 may include software, internal or external storage media and a timing circuit to activate one or more of the actuators 120 after a predetermined time period or time intervals to cause a local increase in the temperature which causes the activation material 116 to expand and which then causes material of interest 102, such as a drug, to be delivered from the microfluidic device 100, 100'.

The system 500 may include a processor 510, which generally provides an execution platform for executing software for controlling the actuators 120. The system 500 also includes memory 520, which may include internal, external, fixed, removable or programmable storage.

A user may interface with the system 500 with one or more input devices 530, such as a keyboard, a mouse, a stylus and the like. The user may also interface with the system 500 with a display 540. A network interface 550, such as telephone, IR or other bus types, may be provided for communicating with other data storage, retrieval and analysis systems. One or more components of the system 500 may be considered optional, such as the display and input device, and other types of components may be used or substituted without departing from a scope of the system 500.

It will be appreciated that the microfluidic devices described may be provided with the power source 124 and the controller 122 integrally formed with the microfluidic devices, or the microfluidic devices may be provided for use with a remote power source 124 and/or a remote controller 122, and in cases where the microfluidic devices are supplied for use with a remote controller 122, a suitable interface would be provided on the microfluidic device for interfacing with the remote controller 122.

What has been described and illustrated herein are embodiments along with some of their variations. The terms, descriptions and figures used herein are set forth by way of illustration only and are not meant as limitations.

## Claims

1. A microfluidic device for controllably moving a material of interest, said microfluidic device comprising a holding cavity (108) for holding the material of interest (102), an activation material (116) configured to expand, wherein expansion of the activation material (116) decreases the size of the holding cavity (108) to thereby cause the material of interest (102) to be released from the holding cavity (108), and at least one actuator (120) configured to induce the activation material to expand, **characterised in that** the activation material (116) comprises a gas configured to remain in a dissolved state at relatively lower temperatures and to evolve back into a gaseous state at relatively higher temperatures, and the at least one actuator (120) is operable on the activation material (116) at multiple levels between a zero induction level whereby the activation material (116) is maintained in the dissolved state, and a maximum induction level to controllably cause the activation material (116) to evolve from the dissolved state to the gaseous state to decrease the size of the holding cavity (108) to release a specified volume of the material of interest (102) therefrom.

2. A microfluidic device as claimed in Claim 1 **characterised in that** the at least one actuator (120) is configured to increase the temperature of the activation material (116) to evolve the activation material (116) back into the gaseous state and thereby cause the material of interest (102) to be released from the holding cavity (108).

3. A microfluidic device as claimed in Claim 1 or 2 further comprising an actuation cavity (114) housing the activation material (116), wherein the actuation cavity (114) is separated from the holding cavity (108) by a flexible membrane (110).

4. A microfluidic device as claimed in Claim 1 or 2 **characterised in that** the material of interest (102) is interspersed with the activation material (116).

5. A microfluidic device as claimed in Claim 4 **characterised in that** expansion of the activation material (116) causes the material of interest (102) and the activation material (116) to be released from the holding cavity (108).

6. A microfluidic device as claimed in Claim 4 or 5 **characterised in that** the material of interest (102) is substantially coated with a substance configured to substantially separate the material of interest (102) from the activation material (116).

7. A microfluidic device as claimed in any preceding claim further comprising a power source (124) for powering the at least one actuator (120), and a controller (122) for controlling delivery of power to the at least one actuator (120), the power source (124) and the controller (122) being integrally formed with the microfluidic device.

8. A microfluidic device as claimed in any preceding claim **characterised in that** the at least one actuator (120) comprises a resistive element configured to become heated through application of a potential difference.

9. A microfluidic device as claimed in any preceding claim further comprising at least one delivery orifice (104) configured for fluid communication with the holding cavity (108).

10. A microfluidic device as claimed in Claim 9 **characterised in that** the at least one delivery orifice (104) comprises a hydrophobic needle.

11. A microfluidic device as claimed in Claim 9 or 10 further comprising at least one of a barrier (107) positioned between the at least one delivery orifice (104) and the holding cavity (108), the barrier (107) being adapted to accommodate the material of interest (102) to flow therethrough when the activation material (116) expands.

12. A microfluidic device as claimed in any preceding claim further comprising the material of interest (102), the material of interest (102) being provided as one of
a form which is soluble in a suitable solvent,
a reactant,
in a pure form,
a form to be reconstituted in the holding cavity (102),
a form to be reconstituted by a solvent,
a form to be reconstituted by contact with a solvent,
a form to be reconstituted by mixing with a solvent, and
a freeze-dried form.

13. A method for delivering a material of interest (102) from a microfluidic device having at least one cavity (114) and a delivery orifice (104), said method comprising:
dissolving a gaseous activation material (116) at a relatively low temperature:
inserting the dissolved gaseous activation material (116) into the at least one cavity (114) of the microfluidic device;
maintaining the dissolved gaseous activation material (116) at a relatively low temperature; and
heating the dissolved gaseous activation material (116) to cause the dissolved gaseous activation material (116) to evolve into a gaseous state and expand, expansion of the gaseous activation material (116) causing the material of interest (102) to be delivered out of the delivery orifice (104).

14. A method as claimed in Claim 13 **characterised in that** the microfluidic device includes a holding cavity (108) and an actuation cavity (114) separated by a flexible membrane (110), and inserting the dissolved gaseous activation material (116) comprises inserting the dissolved gaseous activation material (116) into the actuation cavity (114).

15. A method as claimed in Claim 13 further comprising combining the material of interest (102) and the dissolved gaseous activation material (116) into a mixture, and inserting the mixture into the at least one cavity (114).

## Patentansprüche

1. Mikrofluidische Vorrichtung zur steuerbaren Bewegung eines interessierenden Materials, die mikrofluidische Vorrichtung umfassend einen Aufnahmeraum (108) zur Aufnahme des interessierenden Materials (102), ein Aktivierungsmaterial (116), das ausgebildet ist zu expandieren, wobei die Expansion des Aktivierungsmaterials (116) die Abmessung des Aufnahmeraums (108) verringert, um dadurch auf das interessierende Material (102) einzuwirken, aus dem Aufnahmeraum (108) herausgelassen zu werden, und mindestens einen Aktuator (120), der ausgebildet ist, das Aktivierungsmaterial zur Expansion zu veranlassen, **dadurch gekennzeichnet, dass** das Aktivierungsmaterial (116) ein Gas umfasst, das ausgebildet ist, bei relativ gesehen niedrigeren Temperaturen in einem gelöstem Zustand zu verweilen und bei relativ gesehen höheren Temperaturen in einen gasförmigen Zustand zurückzugehen, und der mindestens eine Aktuator (120) an dem Aktivierungsmaterial (116) gemäß einer Vielzahl von Pegeln zwischen einem Null-Einwirkungs-Pegel, bei dem das Aktivierungsmaterial (116) in dem gelösten Zustand gehalten wird, und einem Maximal-Einwirkungs-Pegel betreibbar ist, um auf das Aktivierungsmaterial (116) steuerbar einzuwirken, vom gelösten Zustand in den gasförmigen Zustand überzugehen, damit die Abmessung des Aufnahmeraums (108) verringert wird, um ein bestimmtes Volumen des interessierenden Materials (102) daraus herauszulassen.

2. Mikrofluidische Vorrichtung nach Anspruch 1, **dadurch gekennzeichnet, dass** der mindestens eine Aktuator (120) ausgebildet ist, die Temperatur des Aktivierungsmaterials (116) zu erhöhen, um das Aktivierungsmaterial (116) in den gasförmigen Zustand zurückzuführen und dadurch auf das interessierende Material (102) einzuwirken, aus dem Aufnahmeraum (108) herausgelassen zu werden.

3. Mikrofluidische Vorrichtung nach Anspruch 1 oder 2, ferner umfassend einen das Aktivierungsmaterial (116) umhausenden Aktivierungsraum (114), wobei der Aktivierungsraum (114) von dem Aufnahmeraum (108) durch eine flexible Membran (110) separiert ist.

4. Mikrofluidische Vorrichtung nach Anspruch 1 oder 2, **dadurch gekennzeichnet, dass** das interessierende Material (102) mit dem Aktivierungsmaterial (116) durchsetzt ist.

5. Mikrofluidische Vorrichtung nach Anspruch 4, **dadurch gekennzeichnet, dass** die Expansion des Aktivierungsmaterials (116) auf das interessierende Material (102) und das Aktivierungsmaterial (116) einwirkt, aus dem Aufnahmeraum (108) herausgelassen zu werden.

6. Mikrofluidische Vorrichtung nach Anspruch 4 oder 5, **dadurch gekennzeichnet, dass** das interessierende Material (102) im Wesentlichen mit einer Substanz beschichtet ist, die ausgebildet ist, das interessierende Material (102) von dem Aktivierungsmaterial (116) im Wesentlichen zu separieren.

7. Mikrofluidische Vorrichtung nach einem der vorhergehenden Ansprüche, ferner umfassend eine Energiequelle (124) zur Energieversorgung des mindestens einen Aktuators (120) und eine Steuerung (122) zum Steuern der Energiezufuhr zu dem mindestens einen Aktuator (120), wobei die Energiequelle (124) und die Steuerung (122) integral mit der mikrofluidischen Vorrichtung ausgebildet sind.

8. Mikrofluidische Vorrichtung nach einem der vorhergehenden Ansprüche, **dadurch gekennzeichnet, dass** der mindestens eine Aktuator (120) ein Widerstandselement umfasst, das ausgebildet ist, durch Anwenden einer Potentialdifferenz geheizt zu werden.

9. Mikrofluidische Vorrichtung nach einem der vorhergehenden Ansprüche, ferner umfassend mindestens eine Förderöffnung (104), die für eine Fluidverbindung mit dem Aufnahmeraum (108) ausgebildet ist.

10. Mikrofluidische Vorrichtung nach Anspruch 9, **dadurch gekennzeichnet, dass** die mindestens eine Förderöffnung (104) eine hydrophobische Nadel umfasst.

11. Mikrofluidische Vorrichtung nach Anspruch 9 oder 10, ferner umfassend mindestens eine zwischen der mindestens einen Förderöffnung (104) und dem Aufnahmeraum (108) angeordneten Barriere (107), wobei die Barriere (107) dazu eingerichtet ist, das interessierende Material (102) derart unterzubringen, dass es hierdurch hindurchfließt, wenn das Aktivierungsmaterial (116) expandiert.

12. Mikrofluidische Vorrichtung nach einem der vorhergehenden Ansprüche, ferner umfassend das interessierende Material (102), wobei das interessierende Material (102) vorgesehen ist als:
eine Ausformung, welche in einem geeigneten Lösungsmittel lösbar ist,
ein Reaktionsmittel,
in einer reinen Form,
eine in dem Aufnahmeraum (102) wiederherzustellende Ausformung,
eine durch ein Lösungsmittel wiederherzustellende Ausformung,
eine durch Kontakt mit einem Lösungsmittel wiederherzustellende Ausformung,
eine durch Mischen mit einem Lösungsmittel wiederherzustellende Ausformung, oder
eine gefriergetrocknete Ausformung.

13. Verfahren zur Förderung eines interessierenden Materials (102) von einer mikrofluidischen Vorrichtung mit mindestens einem Raum (114) und einer Förderöffnung (104), das Verfahrend umfassend:
Lösen eines gasförmigen Aktivierungsmaterials (116) bei einer relativ gesehen niedrigen Temperatur;
Einführen des gelösten gasförmigen Aktivierungsmaterials (116) in den mindestens einen Raum (114) der mikrofluidischen Vorrichtung;
Aufrechterhalten des gelösten gasförmigen Aktivierungsmaterials (116) bei einer relativ gesehen niedrigen Temperatur; und
Heizen des gelösten gasförmigen Aktivierungsmaterials (116), um auf das gelöste gasförmige Aktivierungsmaterial (116) einzuwirken, in einen gasförmigen Zustand überzugehen und zu expandieren, wobei die Expansion des gasförmigen Aktivierungsmaterials (116) auf das interessierende Material (102) einwirkt, um aus der Förderöffnung (104) gefördert zu werden.

14. Verfahren nach Anspruch 13, **dadurch gekennzeichnet, dass** die mikrofluidische Vorrichtung einen Aufnahmeraum (108) und einen Aktivierungsraum (114) aufweist, welche durch eine flexible Membran (110) getrennt sind, und das Einführen des gelösten gasförmigen Aktivierungsmaterials (116) ein Einführen des gelösten gasförmigen Aktivierungsmaterials (116) in den Aktivierungsraums (114) umfasst.

15. Verfahren nach Anspruch 13, ferner umfassend das Kombinieren des interessierenden Materials (102) und des gelösten gasförmigen Aktivierungsmaterials (116) in eine Mischung, und Einführen der Mischung in den mindestens einen Raum (114).

## Revendications

1. Dispositif microfluidique destiné à déplacer en mode asservi un matériau d'intérêt, ledit dispositif microfluidique comprenant une cavité de maintien (108) destinée à maintenir le matériau d'intérêt (102), un matériau d'activation (116) configuré pour se dilater, où la dilatation du matériau d'activation (116) diminue la taille de la cavité de maintien (108) amenant ainsi le matériau d'intérêt (102) à se dégager de la cavité de maintien (108), et au moins un actionneur (120) configuré pour induire la dilatation du matériau d'activation, **caractérisé en ce que** le matériau d'activation (116) comprend un gaz configuré pour rester dans un état dissous à des températures relativement basses et pour revenir à un état gazeux à des températures relativement élevées, et l'au moins un actionneur (120) peut fonctionner sur le matériau d'activation (116) à plusieurs niveaux entre un niveau d'induction nul par lequel le matériau d'activation (116) est maintenu dans l'état dissous, et un niveau d'induction maximal pour amener le matériau d'activation (116) en mode asservi à évoluer de l'état dissous à l'état gazeux pour diminuer la taille de la cavité de maintien (108) afin d'en libérer un volume spécifié du matériau d'intérêt (102).

2. Dispositif microfluidique tel que revendiqué dans la revendication 1 **caractérisé en ce que** l'au moins un actionneur (120) est configuré pour augmenter la température du matériau d'activation (116) afin de faire revenir le matériau d'activation (116) vers l'état gazeux et amener ainsi le matériau d'intérêt (102) à se dégager de la cavité de maintien (108).

3. Dispositif microfluidique tel que revendiqué dans la revendication 1 ou 2 comprenant en outre une cavité d'actionnement (114) recevant le matériau d'activation (116), où la cavité d'actionnement (114) est séparée de la cavité de maintien (108) par une membrane souple (110).

4. Dispositif microfluidique tel que revendiqué dans la revendication 1 ou 2 **caractérisé en ce que** le matériau d'intérêt (102) est entrecoupé du matériau d'activation (116).

5. Dispositif microfluidique tel que revendiqué dans la revendication 4 **caractérisé en ce que** la dilatation du matériau d'activation (116) amène le matériau d'intérêt (102) et le matériau d'activation (116) à se dégager de la cavité de maintien (108).

6. Dispositif microfluidique tel que revendiqué dans la revendication 4 ou 5 **caractérisé en ce que** le matériau d'intérêt (102) est essentiellement revêtu d'une substance configurée pour séparer sensiblement le matériau d'intérêt (102) du matériau d'activation (116).

7. Dispositif microfluidique tel que revendiqué dans l'une des revendications précédentes comprenant en outre un bloc d'alimentation (124) destiné à alimenter l'au moins un actionneur (120), et une unité de commande (122) destinée à commander la distribution de l'énergie vers l'au moins un actionneur (120), le bloc d'alimentation (124) et l'unité de commande (122) étant formés en un seul bloc avec le dispositif microfluidique.

8. Dispositif microfluidique tel que revendiqué dans l'une des revendications précédentes **caractérisé en ce que** l'au moins un actionneur (120) comprend un élément résistif configuré pour être chauffé grâce à l'application d'une différence de potentiel.

9. Dispositif microfluidique tel que revendiqué dans l'une des revendications précédentes comprenant en outre au moins un orifice de distribution (104) configuré pour être en communication fluidique avec la cavité de maintien (108).

10. Dispositif microfluidique tel que revendiqué dans la revendication 9 **caractérisé en ce que** l'au moins un orifice de distribution (104) comprend une aiguille hydrophobe.

11. Dispositif microfluidique tel que revendiqué dans la revendication 9 ou 10 comprenant en outre au moins l'une d'une barrière (107) positionnée entre l'au moins un orifice de distribution (104) et la cavité de maintien (108), la barrière (107) étant adaptée pour recevoir le matériau d'intérêt (102) pour qu'il circule à travers celle-ci lorsque le matériau d'activation (116) se dilate.

12. Dispositif microfluidique tel que revendiqué dans l'une des revendications précédentes comprenant en outre le matériau d'intérêt (102), le matériau d'intérêt (102) étant pourvu comme étant l'un parmi :
une forme qui est soluble dans un solvant approprié,
un réactif,
sous forme pure,
une forme à reconstituer dans la cavité de maintien (102),
une forme à reconstituer par un solvant,
une forme à reconstituer par contact avec un solvant,
une forme à reconstituer par mélange avec un solvant, et
une forme lyophilisée.

13. Procédé de distribution d'un matériau d'intérêt (102) à partir d'un dispositif microfluidique comprenant au moins une cavité (114) et un orifice de distribution (104), ledit procédé comprenant le fait :
de dissoudre un matériau d'activation gazeux (116) à une température relativement basse ;
d'insérer le matériau d'activation gazeux dissous (116) dans l'au moins une cavité (114) du dispositif microfluidique ;
de maintenir le matériau d'activation gazeux dissous (116) à une température relativement basse ; et
de chauffer le matériau d'activation gazeux dissous (116) pour amener le matériau d'activation gazeux dissous (116) à évoluer vers un état gazeux et à se dilater, la dilatation du matériau d'activation gazeux (116) amenant le matériau d'intérêt (102) à être distribuer à l'extérieur de l'orifice de distribution (104).

14. Procédé tel que revendiqué dans la revendication 13 **caractérisé en ce que** le dispositif microfluidique comporte une cavité de maintien (108) et une cavité d'actionnement (114) séparées par une membrane souple (110), et l'insertion du matériau d'activation gazeux dissous (116) comprend l'insertion du matériau d'activation gazeux dissous (116) dans la cavité d'actionnement (114).

15. Procédé tel que revendiqué dans la revendication 13 comprenant en outre le fait de combiner le matériau d'intérêt (102) et le matériau d'activation gazeux dissous (116) dans un mélange, et d'insérer le mélange dans l'au moins une cavité (114).
